(19) Europäisches Patentamt
European Patent Office
Office européen des brevets

(11) EP 0 574 313 B1

(12) **FASCICULE DE BREVET EUROPEEN**

(45) Date de publication et mention de la délivrance du brevet: **06.10.1999 Bulletin 1999/40**

(21) Numéro de dépôt: **93401476.2**

(22) Date de dépôt: **10.06.1993**

(51) Int. Cl.$^6$: **C07D 409/12**, C07D 319/18, C07D 495/04, A61K 31/335, A61K 31/38 // (C07D495/04, 333:00, 333:00)

(54) **Pipérazines 1,4-disubstituées pour le traitement des maladies du système nerveux central et des maladies neuro endocriniennes**

1,4-Disubstituierte Piperazine zur Behandlung von Erkrankungen des Zentralnervensystems und von nervenendokrinen Erkrankungen

1,4-Disubstituted piperazines for the treatment of disorders of the central nervous system and neuroendocrine disorders

(84) Etats contractants désignés:
**AT BE CH DE DK ES FR GB GR IE IT LI LU NL PT SE**

(30) Priorité: **12.06.1992 FR 9207065**

(43) Date de publication de la demande:
**15.12.1993 Bulletin 1993/50**

(73) Titulaire: **ADIR ET COMPAGNIE**
**92415 Courbevoie Cédex (FR)**

(72) Inventeurs:
- **Peglion, Jean-Louis**
  **F-78110 Le Vesinet (FR)**
- **Goument, Bertrand**
  **F-78220 Viroflay (FR)**
- **Millan, Mark**
  **F-75017 Paris (FR)**
- **Rivet, Jean-Michel**
  **F-92000 Nanterre (FR)**

(74) Mandataire: **Reverbori, Marcelle**
**ADIR**
**1, rue Carle Hébert**
**92415 Courbevoie Cédex (FR)**

(56) Documents cités:
**EP-A- 0 376 607** **EP-A- 0 490 772**

- **A. BURGER:'Medicinal Chemistry' JOHN WILEY AND SONS, 3.Edition, Partie C, Page 76, NEW YORK, US**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 574 313 B1

## Description

La présente invention a pour objet de nouvelles pipérazines 1,4-disubstituées et les compositions pharmaceutiques les renfermant.

Elle concerne plus particulièrement les pipérazines 1,4-disubstituées de formule (I) :

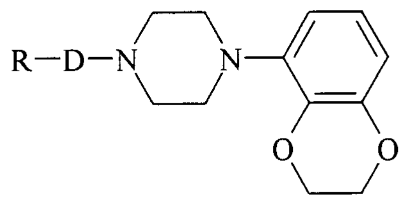

(I)

dans laquelle :

R représente un groupement choisi parmi :

- thiophén-3-yl et, dans ce cas, D représente un groupement choisi parmi méthylène, éthylène, n-propylène, n-butylène et 2,2-diméthyl-n-propylène,
- tétrahydrobenzo[b]thiophén-5yl, cyclopenta[b]thiophén-5-yl et, dans ce cas, D représente le groupement méthylène,
- napht-1-yl, napht-2-yl, thiéno[2,3-b]thiophén-2-yl, thiophén-2-yl et, dans ce cas, D représente le groupement éthylène,
- et phényl et, dans ce cas, D représente le groupement n-propylène,

ainsi que leurs sels physiologiquement tolérables avec des acides appropriés.

L'état antérieur de la technique le plus proche de la présente invention est illustré par les demandes de brevet EP-A-0 376 607 et EP 0 490 772 qui concernent des produits agissant notamment sur le récepteur $5HT_{1A}$ et répondant à la formule :

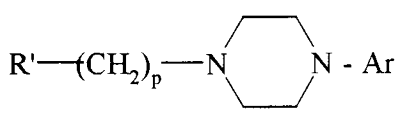

dans laquelle :

- R' représente, entre autre, un radical indolyle,
- p est un entier compris entre 0 et 6 inclus,
- Ar représente, entre autres,

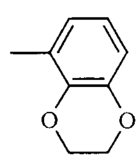

Ces demandes ne suggèrent nullement les composés objet de la présente demande, lesquels diffèrent essentiellement du composé le plus proche de l'art antérieur par les significations de R qui, compte tenu de l'absence de corrélation bio-isostérique intangible entre R et R', ne sauraient être induites par la signification indolyle du groupe R.

Les composés de formule (I) de la présente invention peuvent notamment être obtenus par le procédé de préparation qui consiste au fait que l'on condense :

- la pipérazine N-monosubstituée de formule (II) :

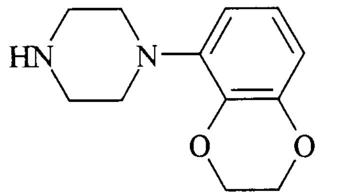

(II)

- ***soit*** *avec :*
  - un composé de formule (III) :

R - D - X (III)

dans laquelle :

  - R et D ont les significations précédemment définies et
  - X représente un atome d'halogène, ou un radical mésyloxy ou tosyloxy,

- ***soit*** *avec :*
  - un composé de formule (IV) :

R - D - COOH (IV)

dans laquelle :

  - R et D ont les significations précédemment définies et l'on réduit l'amide ainsi obtenue de formule (V) :

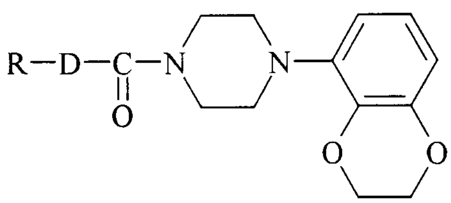

(V)

dans laquelle R et D ont les significations précédemment définies.

La condensation des composés II et III s'effectue de façon particulièrement adéquate en opérant dans un solvant approprié tel que, par exemple, l'acétone, la méthyléthylcétone, la méthylisobutylcétone, le toluène, ou le diméthylformamide en présence d'un accepteur de l'acide formé au cours de la réaction, à une température de 20 à 150°C. Comme accepteur, on peut employer par exemple un carbonate de métaux alcalins comme le carbonate de sodium ou une amine tertiaire comme la triéthylamine.

La condensation des composés II et IV s'effectue de façon particulièrement adéquate en opérant dans un solvant approprié comme par exemple le chlorure de méthylène, en présence de carbonyldiimidazole.

La réduction de l'amide V s'effectue avantageusement au moyen d'un hydrure double de lithium-aluminium

dans un solvant adéquat comme par exemple l'éther ou le tétrahydrofurane.

De plus, les amides de formule V sont des produits intermédiaires nouveaux qui font, à ce titre, partie de la présente invention.

Les matières premières de formules II, III et IV sont soit des produits connus, soit des produits préparés à partir de composés connus selon des procédés connus, comme précisé dans les exemples ci-après.
Les composés de formule I donnent des sels avec les acides physiologiquement tolérables. Ces sels sont également inclus dans la présente invention.
Les composés de la présente invention possèdent des propriétés pharmacologiques et thérapeutiques intéressantes. En effet, les essais pharmacologiques ont démontré que les composés de l'invention se comportent, in vitro et in vivo, comme des ligands très puissants et très sélectifs des récepteurs de la sérotonine $5HT_{1A}$ avec une activité antagoniste de ce neurotransmetteur au niveau du système nerveux central, démontrée par l'étude pharmacologique ci-après exemplifiée.

Cette activité permet l'utilisation des composés de la présente invention dans le traitement des maladies du système nerveux central, notamment des troubles de l'apprentissage et de la mémoire (Carli, M. and Samanin, R. Br. J. Pharmacol., **105**, 720-726, 1992 ; Carli, M., Tranchina, S. and Samanin, R., Eur. j. Pharmacol., 211, 227-234, 1992 ; Lister, R.G., $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp. 267-280, 1991), de l'anxiété (Barrett. J.E., and Gleeson, S. $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp. 59-105, 1991 ; Glennon, R.A., Neurosci. & Behav. Rev. **14** : 35-47, 1990 ; Lader, M.H., $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp 343-363, 1991 ; Schweizer, E., and Rickels, K., $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp 365-376, 1991 ; Taylor, D.P., and Moon, S.L., Neuropeptides, **19** : 15-19, 1991 ; Treit, D., $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp. 107-131, 1991) , la dépression (Cervo, L., Grignaschi, G. and Samanin, R. Eur. J. Pharmacol., **158** : 53-59, 1988 ; Glennon, R. A. Neurosci. & Behav. Rev . **14** : 35-47, 1990 ; Thiébot, M.-H. and Martin, P. $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp. 159-194, 1991), la schizophrénie (Ahlenius, S., Pharmacol & Toxicol., **64** :3-5, 1989 ; Glennon, R.A., Neurosci. & Behav. Rev. **14** : 35-47, 1990 ; Invernizzi, R.W., Cervo, L., and Samanin, R., Neuropharmacology, **27** : 515-518, 1988), le stress (Dourish, C.R., Hutson, P.H., and Ahlenius S., (Eds.) Brain $5\text{-}HT_{1A}$ receptors, Chichester Press, Horwood, England, 1987 ; Fuller, R.W., Neuropsycho-pharmacology **3** : 495-502, 1990 ; Glennon, R.A., neurosci. & Behav. Rev. **14** : 35-47, 1990), l'anorexie (Cooper, S.J., $5\text{-}HT_{1A}$ agonists, $5\text{-}HT_3$ antagonists and benzodiazepines : their comparative behavioural pharmacology Ed. R.J. Rodgers and S.J. Cooper, Wiley & Sons Ltd, Chichester, pp. 233-265, 1991 ; Dourish, C.R., Hutson, P.H., and Ahlenius S., (Eds.) Brain $5\text{-}HT_{1A}$ receptors, Chichester Press, Horwood, England, 1987), la douleur (Berge, O.G., Hole, K., and Dahle, H., Neurosci. Lett., **19** : 219-223, 1980 Daval, G., Vergé, D., Basbaum, A.I., Bourgoin, S., and Hamon, M. Neurosci. Lett., **83** : 71-76, 1987 ; Fasmer, O.B., Berge, O.G., Post, C. and Hole, K., Pharmacol. Biochem. Behav., **25** : 883-888, 1986 ; Hamon, M., Collin, E., Chantrel, D., Daval, G., Vergé, D., Bourgoin, S. and Cesselin, F., Serotonin and Pain, ed. by J.-M. Besson, Elsevier, Amsterdam, pp. 53-72, 1990 ; Millan, M.J., Bervoets K. and Colpaert F.C. J. Pharmacol. Exp. Ther., **256** : 973-982, 1991a ; Millan, M.J., and Colpaert F.C., J. Pharmacol. Exp. Ther., 256 : 983-992, 1991a ; Millan, M.J. and Colpaert F.C., J. Pharmacol. Exp. Ther., 256 : 993-1001, 1991b) et des maladies neuro-endocriennes comme le diabète (Chaouloff, F. and Jeanrenaud, B. J., Pharmacol. Exp. Ther., 243, 1159-1166, 1990 ; Schweizer, E. and Rickels, K., $5\text{-}HT_{1A}$ agonists, $5\text{-}HT_3$ antagonists and benzodiazepines : their comparative behavioural pharmacology Ed. R.J. Rodgers and S.J. Cooper, Wiley & Son Ltd, Chichester, pp. 365-376- 1991 ; Taylor , D.P. and Moon, S.L., Neuropeptides, **19** : 15-19, 1991). Une implication (par exemple l'hyperactivité) des récepteurs $5\text{-}HT_{1A}$ a été clairement mise en évidence dans ces troubles (voir références ci-dessus). De surcroît, l'efficacité du ligand $5\text{-}HT_{1A}$, la buspirone, dans le traitement de certaines de ces maladies (par exemple l'anxiété) a été montrée chez l'homme (Goff, D.C., Midha, K.K., Brotman, A.W., McCormick, S., Waites, M. and Amico, E.T., J. Clin. Psychopharmacol., **11** : 193-197, 1991). Les résultats obtenus lors de l'étude pharmacologique des composés de la présente invention montrent sans la moindre ambiguité que ces dits composés sont actifs sur les récepteurs $5\text{-}HT_{1A}$ in vitro et in vivo, et de ce fait sont utilisables pour la thérapie des troubles mentionnés ci-dessus.

La présente invention a également pour objet les compositions pharmaceutiques contenant comme principe actif un composé de formule I ou un de ses sels physiologiquement tolérables, mélangé ou associé à un excipient pharmaceutique approprié, comme par exemple, le glucose, le lactose, le talc, l'éthylcellulose, le stéarate de magnésium ou le beurre de cacao.

Les compositions pharmaceutiques ainsi obtenues se présentent généralement sous forme dosée et peuvent contenir de 0,1 à 100 mg de principe actif. Elles peuvent revêtir, par exemple, la forme de comprimés, dragées, gélules, suppositoires, solutions injectables ou buvables et être selon les cas, administrées par voie orale, rectale ou parentérale à la dose de 0,1 à 100 mg de principe actif 1 à 3 fois par jour.

Les exemples suivants illustrent la présente invention, les points de fusion étant déterminés à la platine chauffante de Kofler (K) éventuellement sous microscope (M.K).

## Exemple 1

4-(Benzodioxan-5-yl)-1-[3-(thiophén-3-yl)propyl]pipérazine :

Stade 1

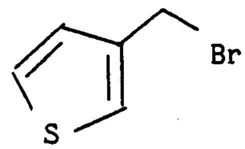

On coule goutte à goutte en 20 minutes 7,05 ml (75,0 mmol.) de tribromure de phosphore sur 25,4 g (222,5 mmol.) de thién-3-yl méthanol en solution dans 45 ml de benzène vers 2-3 °C. On maintient 1 heure sous agitation à cette température, puis 4 heures à température ambiante. On verse le milieu réactionnel dans un mélange eau/glace, puis extrait à l'éther. Les phase éthérées jointes sont lavées à l'eau. Après séchage sur sulfate de magnésium et concentration, puis distillation au Kugelrohr (Eb. : 40-80 °C sous 1866 à 1999 Pa), on recueille 30,2 g du composé bromé attendu (Rendement : 77 %).

Stade 2

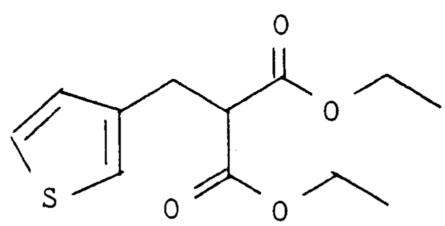

A température ambiante, sur une solution d'éthylate de sodium préparée à partir de 4,17 g (181,2 m.atm.g) de sodium et 100 ml d'éthanol anhydre, on coule goutte à goutte en 15 minutes 29,0 g (181,2 mmol.) de malonate de diéthyle. On maintient 1 heure l'agitation à température ambiante, puis coule goutte à goutte en 1 heure 15, le composé bromé préparé au stade 1 en maintenant la température à environ 16 °C. On maintient encore 1 heure sous agitation à température ambiante, puis 1 heure à reflux. On évapore à sec, reprend à l'eau et extrait à l'éther. Après séchage sur sulfate de magnésium et concentration, puis distillation au Kugelrohr (Eb. : 50-100 °C sous 6,664 Pa), on recueille 28,6 g du diester. (Rendement : 66 %).

Stade 3

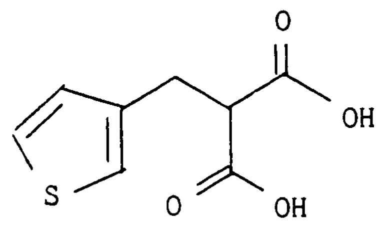

A 28,3g (110,4 mmol.) du diester précédent dans 55 ml d'éthanol à température ambiante, on ajoute 24,8 g (441,6 mmol.) de potasse dans 25 ml d'eau, puis porte à reflux 7 heures. Après évaporation à sec, on reprend à l'eau et verse lentement dans 90 ml d'acide chlorhydrique 6N. On extrait à l'acétate d'éthyle. Les phases organiques jointes sont lavées par une solution aqueuse saturée de chlorure de sodium. Après séchage sur sulfate de magnésium et concentration, on recueille 20,4 g du diacide. (Rendement : 92 %). PF (K) : 138 °C.

Stade 4

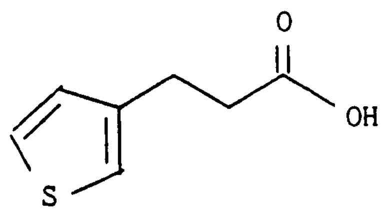

On dissout 20,3 g (101,4 mmol.) du diacide précédent dans 100 ml de N,N-diméthylacétamide, puis porte 1 heure à reflux. Après évaporation du solvant, on reprend à l'éther et lave à l'eau. Après séchage sur sulfate de magnésium et concentration, on recueille 14,2 g de l'acide attendu. (Rendement : 90 %). PF (K) : environ 50 °C.

Stade 5

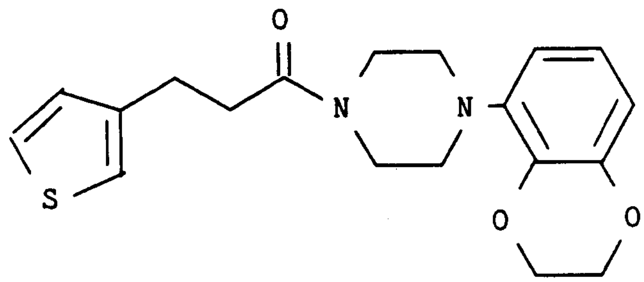

A 3,13 g (20,0 mmol.) de l'acide précédent dans 40 ml de chlorure de méthylène à température ambiante, on ajoute d'un trait 3,41 g (21,0 mmol.) de carbonyl-diimidazole. On maintient 7 heures sous agitation à température

ambiante, puis ajoute une solution de 4,4 g (20 mmol.) de N-(benzodioxan-5-yl)-pipérazine dans 25 ml de chlorure de méthylène. On maintient encore la nuit sous agitation à température ambiante, puis ajoute du chlorure de méthylène et lave avec de la soude N, puis de l'eau. Après séchage sur sulfate de magnésium et concentration, on recueille 7,4 g de l'amide attendu sous forme d'une huile. (Rendement quantitatif).

Stade 6

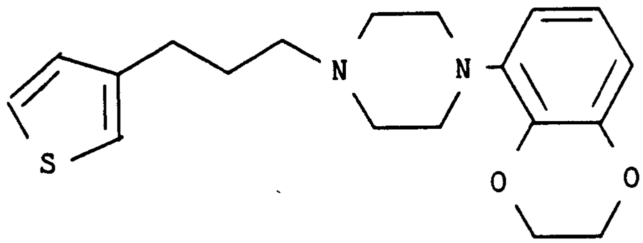

A 0,76 g (20,0 mmol.) d'aluminohydrure de lithium dans 40 ml de THF à température ambiante, on ajoute goutte à goutte, en 1 heure 10, 7,4 g (20,0 mmol.) de l'amide précédent dans 130 ml de THF. On maintient la nuit sous agitation à température ambiante, puis hydrolyse à 0 °C par 0,53 ml d'eau, puis 0,42 ml de soude à 20 %, puis 1,9 ml d'eau. On filtre les sels formés sur fritté. Après séchage sur sulfate de magnésium et concentration, on recueille 7,0 g du produit brut.
Celui-ci est solubilisé dans 100 ml d'éthanol, puis traité par 2,2 éq. d'éther chlorhydrique.
Après évaporation à sec et recristallisation dans 40 ml d'éthanol, on obtient 5,1 g du dichlorhydrate impur.
Après retour à la base (4,2 g) et recristallisation dans 21 ml d'éther isopropylique, on obtient 2,5 g de 4-(benzodioxan-5-yl)-1-[3-(thiophén-3-yl)propyl]-pipérazine. (Rendement : 32 %).

PF (K) : 71-73 °C.
RMN : $^1H$ (CDCl3/TMS) : 7,25(m,1H) ; 6,95 (m,2H) ; 6,75 (t,1H) ; 6,55 (m,2H) ; 4,25 (m,4H) ; 3,10 (m,4H) ; 2,75 (m,6H) ; 2,45 (m,2H) ; 1,85 (m,2H).

**Exemple 2** :

4-(Benzodioxan-5-yl)-1-[(4,5,6,7-tétrahydro benzo[b]thiophén-5-yl)méthyl] pipérazine (R,S) , et son dichlorhydrate.

Stade 1

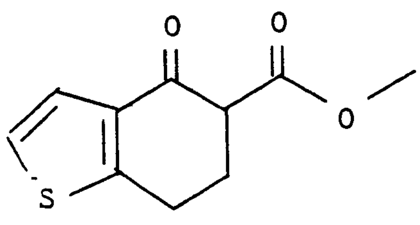

A 23,6 g (155 mmol.) de 4-oxo cyclohexa[b]thiophène (produit commercial) dans 475 ml de carbonate de diméthyle à 0 °C, on ajoute par fractions en 10 minutes 16,6 g (465 mmol.) d'hydrure de sodium à 60 %. On porte à reflux sous agitation énergique pendant 3 heures. On laisse refroidir et verse sur 1 l d'un mélange de glace et d'eau contenant 45 ml d'acide acétique. On extrait à l'éther. Après séchage sur sulfate de magnésium et concentration, puis recristallisation dans 150 ml d'éther isopropylique, on recueille 20,4 g du cétoester attendu. (Rendement : 94 %).

PF (K) : 88 °C.

Stade 2

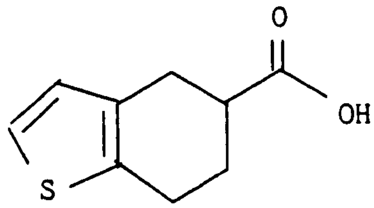

On prépare l'amalgame de zinc à partir de 69,1 g de zinc, 6,9 g de chlorure mercurique, 3,2 ml d'acide chlorhydrique concentré et 103 ml d'eau. Après avoir décanté la phase aqueuse, on rajoute 55 ml d'eau, 116 ml d'acide chlorhydrique concentré et 30,4 g (144,6 mmol.) du cétoester précédent dans 72 ml de toluène. On porte à reflux 2 heures, puis laisse refroidir et sépare les phases. La phase aqueuse est réextraite au toluène. Les phase toluéniques jointes sont extraites avec une solution aqueuse saturée d'hydrogénocarbonate de sodium, puis lavées à l'eau. Après séchage sur sulfate de magnésium et concentration, puis distillation au Kugelrohr (Eb.: 55-75 °C sous 40 Pa), on recueille 20,5 g de l'ester méthylique correspondant à l'acide attendu. (Rendement : 78 %).
Les phases aqueuses hydrogénocarbonate de sodium sont jointes et coulées goutte à goutte à 0 °C sur une solution d'acide chlorhydrique 6N. On filtre le précipité formé et le lave à l'eau. Après séchage à l'air, on obtient 2,25 g de l'acide attendu. (Rendement : 8 %).

PF (K) : 115 °C.

Stade 3

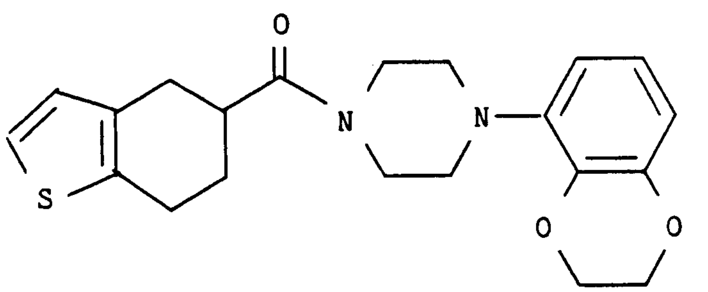

On procède comme dans l'exemple 1 stade 5 à partir de 2,3 g (12,6 mmol.) de l'acide précédent et 2,8 g (12,6 mmol.) de N-(benzodioxan-5-yl)-pipérazine. On recueille 4,8 g de l'amide attendu sous forme d'une meringue. (Rendement quantitatif).

Stade 4

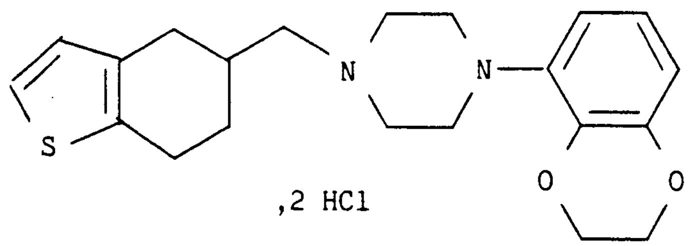

On procède comme dans l'exemple 1 stade 6 à partir de 4,6 g de l'amide précédent. Le produit brut obtenu, 4-(benzodioxan-5-yl)-1-[(4-5,6,7-tétrahydrobenzo[b]thiophén-5-yl)méthyl]pipérazine (R,S) est chromatographié sur silice (éluant : dichlorométhane/méthanol 97/3), puis solubilisé dans 100 ml d'éthanol et traité par 2,2 éq. d'éther chlorhydrique. Après évaporation à sec et recristallisation dans 70 ml d'éthanol, on obtient 3,0 g de dichlorhydrate de 4-(benzodioxan-5-yl)-1-[(4,5,6,7-tétrahydro benzo[b]thiophén-5-yl) méthyl]pipérazine(R,S). (Rendement : 56 %).

PF (MK) : 213-217 °C (sublimation vers 180 °C).
RMN : $^1H$ (CDCl3/TMS) : 7,65 (1H) ; 7,05 (d,1H) ; 6,85 à 7,0 (m,2H) ; 6,8 (d,1H) ; 5,15 (m,2H) ; 4,2 à 4,6 (m,6H) ; 3,65 (m,4H) ; 2,75 à 3,3 (m,5H) ; 2,25 à 2,7 (m,3H) ; 1,75 (m,1H) ; 13,4 (s large échangeable par $D_2O$).

**Exemple 3:**

4-(Benzodioxan-5-yl)-1-[(cyclopenta[b]thiophén-5-yl)méthyl]pipérazine(R,S)

Stade 1

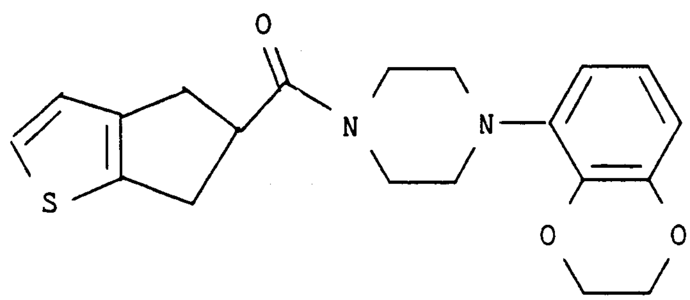

On procède comme dans l'exemple 1 stade 5, à partir de 2,6 g (11,9 mmol.) de N-(benzodioxan-5-yl)-pipérazine et de 2,0 g (11,9 mmol.) d'acide cyclopenta[b]thiophène 5-carboxylique, lui-même obtenu comme décrit dans l'exemple 2 stades 1 et 2 à partir de 6-oxo cyclopenta[b]thiophène (dont la synthèse est décrite dans : J. Pharm. Sciences 1963, 52, 898). On recueille 3,7 g de l'amide sous forme d'une meringue (Rendement : 84 %), après filtration du produit brut sur silice (éluant dichlorométhane/méthanol 97,5/2,5).

Stade 2

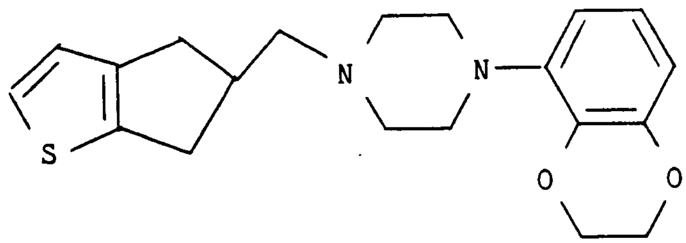

On procède comme dans l'exemple 1 stade 6, à partir de 3,6 g de l'amide précédemment obtenu. Le produit brut obtenu est chromatographié sur silice (éluant : acétate d'éthyle), puis solubilisé dans 50 ml d'éthanol et traité par 2,2 éq. d'éther chlorhydrique. Le dichlorhydrate formé est purifié par retour à la base et recristallisation dans 35 ml d'éther isopropylique ; on obtient 0,60 g de 4-(benzodioxan-5-yl)-1-[(cyclopenta[b]thiophén-5-yl)méthyl]pipérazine(R,S) (base libre). (Rendement : 17 %).

PF(MK) : 146-147 °C
RMN : $^1$H(CDCl3/TMS) : 7,15 (d,1H) ; 6,75 (m,2H) ; 6,55 (m,2H) ; 4,25 (m,4H) ; 2,8 à 3,3 (m,7H) ; 2,4 à 2,75 (m,8H).

**Exemple 4:**

4-(Benzodioxan-5-yl)-1-[4-(thiophén-3-yl)butyl]pipérazine, et son dichlorhydrate

Stade 1

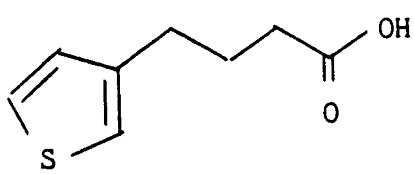

L'acide 4-(3-thiényl)butanoïque a été obtenu par synthèse malonique à partir du 2-(3-thiényl)bromoéthane selon les méthodes décrites dans l'exemple 1 stades 1, 2, 3 et 4.

Stade 2

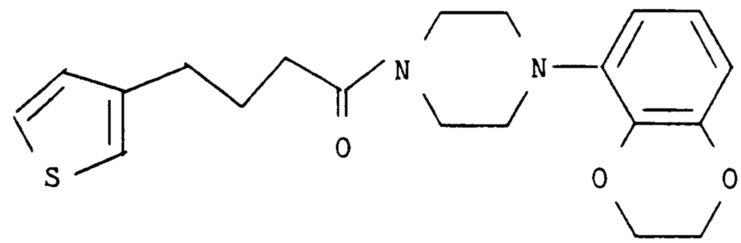

On procède comme dans l'exemple 1 stade 5 à partir de 2,06 g (12,1 mmol) de l'acide précédent. On recueille 4,4 g de l'amide attendu (Rendement : 98 %).

Stade 3

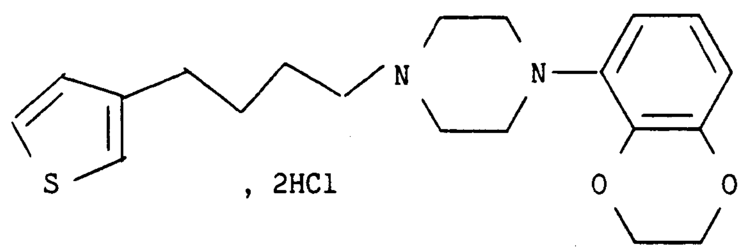

, 2HCl

On procède comme dans l'exemple 1 stade 6 à partir de 4,3 g (11,5 mmol) de l'amide précédent. Après réaction, la 4-(benzodioxan-5-yl)-1-[4-(thiophén-3-yl)butyl]pipérazine obtenue est chromatographiée sur silice (éluant dichlorométhane/méthanol 98/2), puis solubilisée dans 50 ml de méthanol et traitée par 7,8 ml (2,2 éq) d'éther chlorhydrique 2,3 N. Après évaporation et recristallisation dans 15 ml de méthanol, on obtient 2,1 g du dichlorhydrate de 4-(benzodioxan-5-yl)-1-[4-(thiophén-3-yl)butyl] pipérazine, P.F. (M.K) : 210-217 °C (Rendement : 42 %).

**Exemple 5**

4-(Benzodioxan-5-yl)-1-[2-(napht-1-yl)éthyl]pipérazine, et son monochlorhydrate.

Stade 1

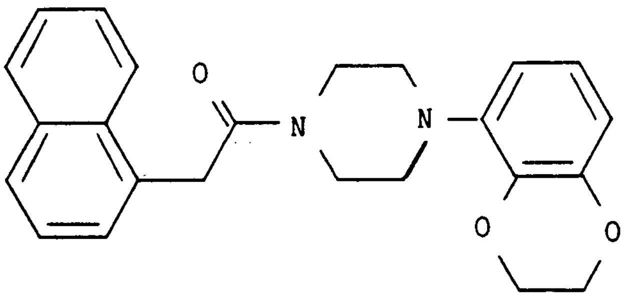

On procède comme dans l'exemple 1 stade 5 à partir de 1,86 g (10,0 mmol) d'acide α-naphtyl acétique commercial. On recueille 3,45 g de l'amide attendu (Rendement : 89 %).

Stade 2

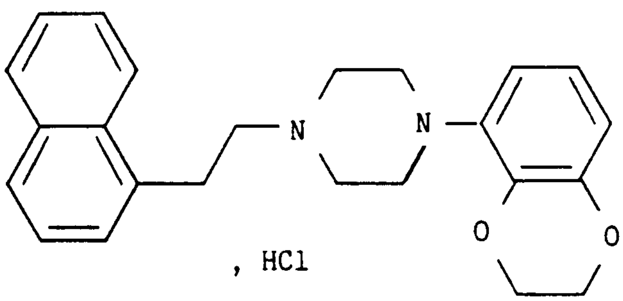

, HCl

On procède comme dans l'exemple 1 stade 6 à partir de 3,4 g (8,7 mmol) de l'amide précédent. Après réaction, la produit obtenu est solubilisé dans 50 ml de méthanol, puis traité par 8,7 ml d'éther chlorhydrique 2,1 N. Après évaporation et recristallisation dans 75 ml de méthanol, on obtient 1,55 g de monochlorhydrate de 4-(benzodioxan-5-yl)-1-[2-(napht-1-yl)éthyl]pipérazine, P.F. (M.K) : 255-260 °C (Rendement: 43 %).

**Exemple 6**

4-(Benzodioxan-5-yl)-1-[2-(napht-2-yl)éthyl]pipérazine et son monochlorhydrate.

Stade 1

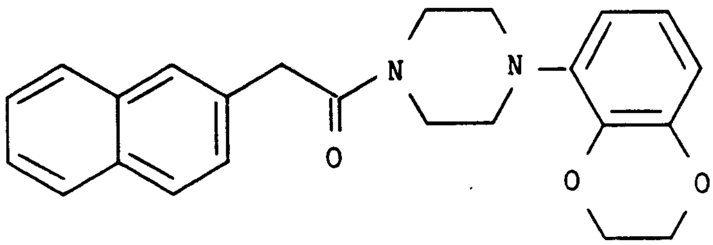

On procède comme dans l'exemple 1 stade 5 à partir de 1,86 g (10,0 mmol) d'acide β-naphtyl acétique commercial. On recueille 3,55 g de l'amide (Rendement : 91 %).

Stade 2

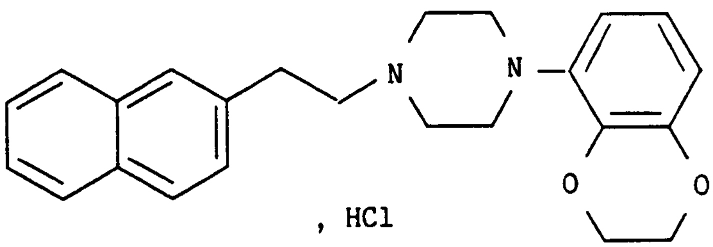

On procède comme dans l'exemple 1 stade 6 à partir de 3,3 g (8,5 mmol) de l'amide précédent. Après réaction, le produit obtenu est recristallisé dans 40 ml d'éther isopropylique, puis solubilisé dans 50 ml de méthanol, puis traité par 4,6 ml d'éther chlorhydrique 2,1 N. Après évaporation et double recristallisation dans le méthanol, on obtient 1,05 g de monochlorhydrate de 4-(benzodioxan-5-yl)-1-[2-(napht-2-yl)éthyl] pipérazine, P.F (M.K) : 249-263 °C (Rendement : 30 %).

**Exemple 7**

4-(Benzodioxan-5-yl)-1-[2-(thiéno[2,3-b]thiophén-2-yl)éthyl]pipérazine

Stade 1

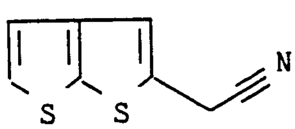

A 5,6 g (32,9 mmol) de 2-hydroxyméthyl thiéno[2,3-b]thiophène (dont la synthèse est décrite dans J. Med Chem. 1991, 34(6), 1805-1817) et 2,7 ml (32,9 mmol) de pyridine dans 66 ml de chloroforme à 0 °C, on ajoute goutte à goutte en 15 mn 2,6 ml (36,2 mmol) de chlorure de thionyle. On agite 2 heures à température ambiante, puis 15 minutes à reflux, puis on refroidit, lave à l'eau et sèche quelques minutes sur sulfate de magnésium, avant de réaliser l'échange de solvant par du toluène. Cette solution toluénique est ajouté goutte à goutte en 55 minutes sur 3,22 g (65,8 mmol) de cyanure de sodium finement broyé en suspension dans 40 ml de DMSO à température ambiante. On maintient 3 jours sous agitation à cette température, puis verse dans de l'eau et extrait à l'éther. Les phases éthérées jointes sont lavées à l'eau, séchées sur sulfate de magnésium et concentrées pour donner 4,6 g du nitrile attendu P.F (K) : 56 °C (Rendement : 78 %).

Stade 2

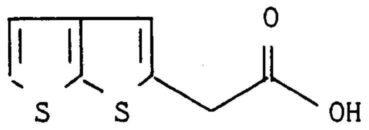

On procède comme au stade 2 de l'exemple 7, à partir de 4,5 g (25,1 mmol) du nitrile du stade 1 ci-dessus décrit, on obtient 3,8 g de l'acide attendu P.F (K) : 138 °C (Rendement : 76 %).

Stade 3

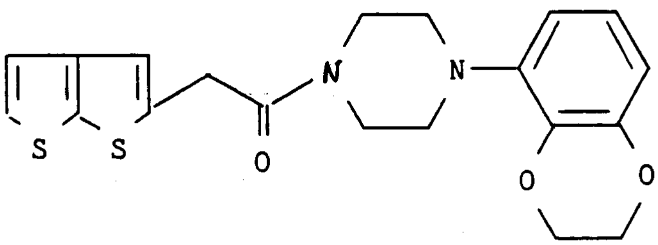

On procède comme dans l'exemple 1 stade 5 à partir de 3,75 g (18,9 mmol) de l'acide obtenu au stade précédent. On obtient, après chromatographie sur silice (éluant : dichlorométhane/méthanol 97/3), 5,55 g de l'amide attendu (Rendement : 73 %).

Stade 4

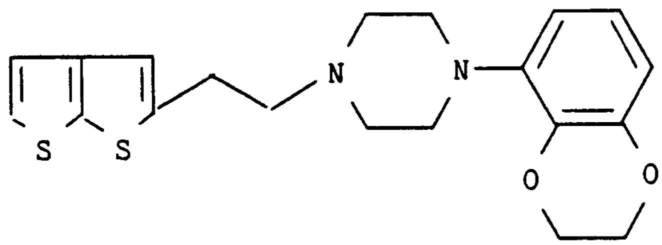

On procède comme dans l'exemple 1 stade 6 à partir de 5,5 g (13,7 mmol) de l'amide obtenu au stade précédent. Après réaction, le produit obtenu est chromatographié sur silice (éluant : dichlorométhane/acétate d'éthyle 50/50), puis recristallisé dans 50 ml de méthanol, puis dans 10 ml d'acétonitrile pour donner 0,90 g de 4-(benzodioxan-5-yl)-1-[2-(thiéno-[2,3-b]thiophén-2-yl)éthyl]pipérazine, P.F (M.K) : 120-122 °C (Rendement : 17 %).

**Exemple 8**

4-(Benzodioxan-5-yl)-1-{[2,2-diméthyl-3-(thiophén-3-yl)]propyl}pipérazine, et son dichlorhydrate

Stade 1

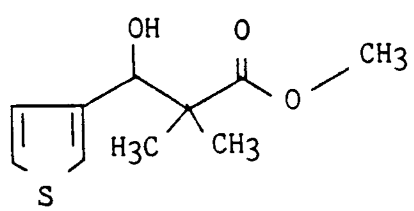

A 11,2 g (100 mmol) de thiophène-3-carboxaldéhyde dans 800 ml de dichlorométhane à -78 °C, on ajoute en 30 minutes 20,9 g (110 mmol)de tétrachlorure de titane dans 220 ml de dichlorométhane. On maintient sous agitation à -78 °C pendant 15 minutes puis ajoute en 5 minutes 19,2 g (110 mmol) de 2-méthyl 1-méthoxy 1-triméthylsilyloxy propène dans 120 ml de dichlorométhane. Après 2 heures à -78 °C, puis 2 heures vers - 60 °C, on hydrolyse à -78 °C par 600 ml d'une solution aqueuse à 10 % de carbonate de potassium. On laisse revenir à température ambiante, puis sépare les phases et réextrait la phase aqueuse avec du dichlorométhane. Les phases organiques jointes sont lavées à l'eau et séchées sur sulfate de magnésium. Après évaporation et concrétisation dans l'hexane, on recueille 10,7 g de l'hydroxy ester attendu P.F (K) : 66 °C (Rendement : 50 %).

Stade 2

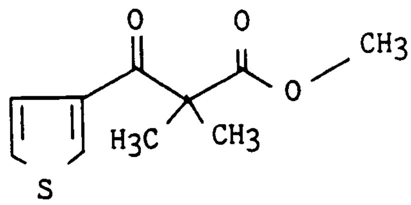

A 5,1 ml d'acide sulfurique concentré dans 60 ml d'eau à 0 °C, on ajoute par fractions 11,0 g (37,4 mmol) de dichromate de potassium, puis 6,4 g (29,9 mmol) de l'hydroxy ester obtenu au stade précédent. On laisse revenir à température ambiante et agite 4 heures. On extrait alors à l'éther, les phases éthérées jointes sont lavées à l'eau jusqu'à neutralité et séchées sur sulfate de magnésium. Après évaporation et chromatographie sur silice (éluant : dichlorométhane/acétate d'éthyle 97/3), on recueille 3,15 g du céto ester attendu, sous forme d'une huile (Rendement : 50 %).

Stade 3

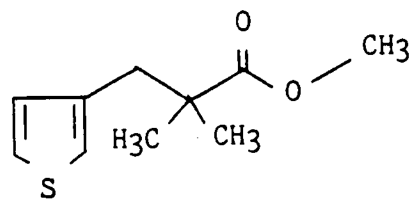

0,68 g (2,5 mmol) de chlorure mercurique et 6,8 g (103 mmol) de poudre de zinc intimement broyés sont agités 15 minutes dans 10 ml d'eau contenant 0,31 ml d'acide chlorhydrique concentré. On élimine la phase liquide et sur l'amalgame, on ajoute 5,6 ml d'eau et 11,3 ml d'acide chlorhydrique concentré, puis 3,0 g (14,1 mmol) du céto ester obtenu au stade précédent dans 10 ml de toluène. On agite énergiquement à reflux pendant 7 heures, puis refroidit et élimine l'amalgame. On sépare les phases et réextrait la phase aqueuse avec du toluène. Les phases toluéniques jointes sont lavées avec une solution aqueuse saturée d'hydrogénocarbonate de sodium, puis à l'eau et séchées sur sulfate de magnésium. Après évaporation et chromatographie sur silice (éluant : dichlorométhane), on recueille 2,0 g de l'ester attendu, sous forme d'huile (Rendement : 71 %).

Stade 4

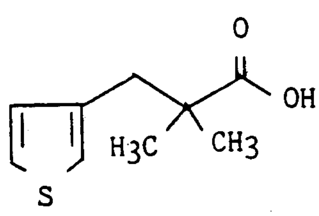

1,9 g (9,6 mmol) de l'ester précédent dans 10 ml de méthanol et 10 ml de soude N sont agités 23 heures à température ambiante. On évapore à sec, puis reprend par 20 ml d'acide chlorhydrique N et extrait à l'acétate d'éthyle. Les phases organiques jointes sont lavées avec une solution aqueuse saturée de chlorure de sodium et séchées sur sulfate de magnésium. Après évaporation, on recueille 1,68 g de l'acide attendu P.F (K) : 56 °C (Rendement : 95 %).

Stade 5

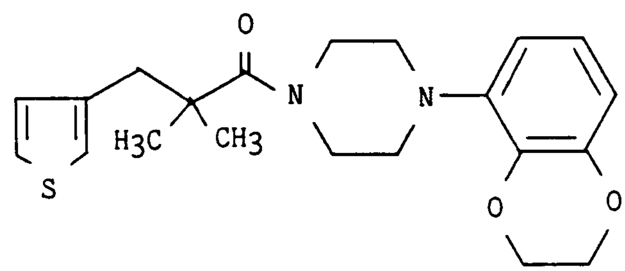

On procède comme dans l'exemple 1 stade 5 à partir de 1,55 g (8,4 mmol) de l'acide obtenu au stade précédent. On obtient, après chromatographie sur silice (éluant : dichlorométhane/méthanol 97/3), 1,3 g de l'amide attendu (Rendement : 40 %).

Stade 6

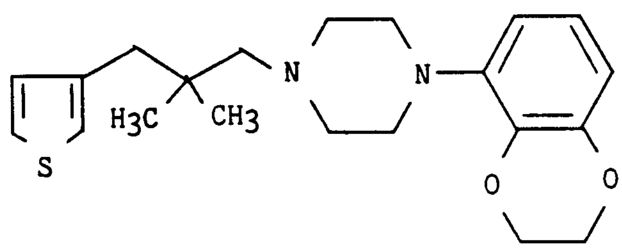

On procède comme dans l'exemple 1 stade 6 à partir de 1,22 g (3,2 mmol) de l'amide obtenu au stade précédent. Après réaction, le produit obtenu est chromatographié sur silice (éluant : dichlorométhane/acétate d'éthyle 50/50), puis concrétisé dans 15 ml d'acétonitrile pour donner 0,19 g de dichlorhydrate de 4-(benzodioxan-5-yl)-1-{[2,2-diméthyl-3-(thiophén-3-yl)]propyl}pipérazine, P.F (M.K) : 201-203 °C.

**Exemple 9**

4-(Benzodioxan-5-yl)-1-[(thiophén-3-yl)méthyl]pipérazine, et son dichlorhydrate

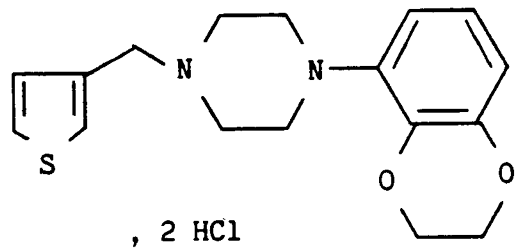

, 2 HCl

A température ambiante, on mélange 1,15 g (6,5 mmol de bromure de (3-thienyl)méthane (obtenu à l'exemple 1 stade 1), 1,6 g (6,2 mmol) de monochlorhydrate de 4-(benzodioxan-5-yl)pipérazine, 2,63 g (24,8 mmol) de carbonate de sodium dans 25 ml de méthyl isobutyl cétone. On porte à reflux 20 heures, puis évapore à sec, reprend à l'acétate d'éthyle et lave par la soude N, puis par une solution aqueuse saturée de chlorure de sodium, puis sèche sur sulfate de magnésium. Après évaporation, puis chromatographie sur silice (éluant : dichlorométhane/méthanol 97/3), le résidu, 4-(benzodioxan-5-yl)-1-[(thiophén-3-yl)méthyl]pipérazine, (1,7 g) est solubilisé dans 25 ml d'éthanol et traité par 5,2 ml (2,2 éq) d'éther chlorhydrique 2,3 N. On évapore à sec, puis recristallise dans 30 ml d'éthanol pour obtenir 1,22 g de dichlorhydrate de 4-(benzodioxan-5-yl)-1-[(thiophén-3-yl)méthyl]pipérazine, P.F (M.K) : 218-223 °C (Rendement : 62 %).

**Exemple 10**

4-(Benzodioxan-5-yl)-1-[2-thiophén-3-yl)éthyl]pipérazine

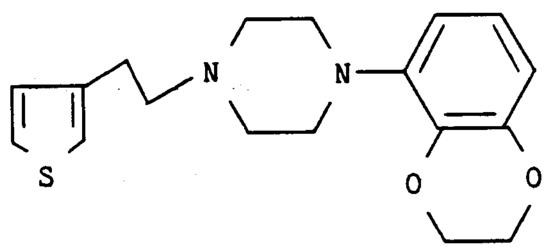

On procède comme dans l'exemple 9 à partir de 1,37 g (6,5 mmol) de 1-bromo 2-(3-thienyl)éthane (obtenu à partir de 2-(3-thiényl)éthanol commercial par la méthode de l'exemple 1 stade 1). Après réaction, le produit obtenu est recristallisé dans 25 ml d'éther isopropylique pour conduire à 1,25 g de 4-(benzodioxan-5-yl)-1-[2-(thiophén-3-yl)éthyl]pipérazine, P.F (M.K) : 100-102°C (Rendement : 61 %).

**Exemple 11**

4-(Benzodioxan-5-yl)-1-(3-phénylpropyl)pipérazine, et son dichlorhydrate

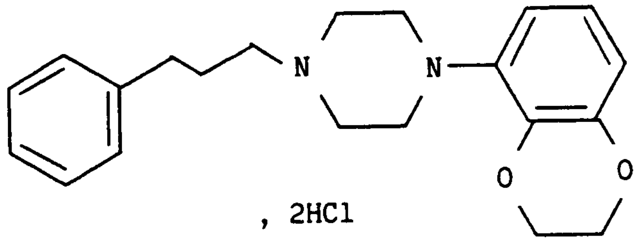

, 2HCl

On procède comme dans l'exemple 10, à partir de 0,91 ml (6,0 mmol) de 1-bromo 3-phényl propane commercial. Après réaction et chromatographie sur silice (éluant : dichlorométhane/méthanol 97/3), le résidu, 4-(benzodioxan-5-yl)-1-(3-phénylpropyl)pipérazine (1,7 g) est solubilisé dans 25 ml d'éthanol et traité par 4,8 ml d'éther chlorhydrique 2,3 N. On évapore à sec, puis recristallise dans 20 ml d'éthanol pour obtenir 1,47 g de dichlorhydrate de 4-(benzodioxan-5-yl)-1-(3-phénylpropyl)pipérazine, P.F (M.K) : 200-203 °C (Rendement : 75 %).

**Exemple 12**

4-(Benzodioxan-5-yl)-1-[2-(thiophén-2-yl)éthyl]pipérazine et son monochlorhydrate

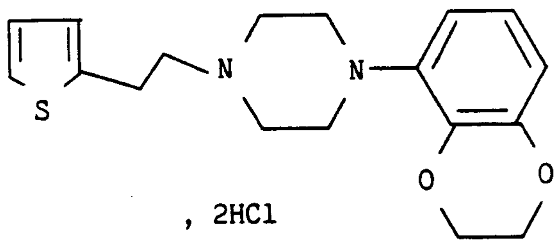

, 2HCl

On procède comme dans l'exemple 9, à partir de 2,53 g (12,0 mmol) de 1-bromo 2-(2-thiényl)éthane (obtenu à partir de 2-(2-thiényl)éthanol commercial par la méthode de l'exemple 1 stade 1). Après réaction, le résidu, 4-(benzodioxan-5-yl)-1-[2-(thiophén-2-yl)éthyl]pipérazine, (2,85 g) est solubilisé dans 50 ml de méthanol, puis traité par 4,9 ml d'éther chlorhydrique 2,1 N. On évapore à sec, puis recristallise dans 35 ml d'éthanol pour obtenir 2,4 g de monochlorhydrate de 4-(benzodioxan-5-yl)-1-[2-(thiophén-2-yl)éthyl]pipérazine, P.F (M.K) : 235-240 °C (Rendement : 60 %).

**Exemple 13**

ETUDE PHARMACOLOGIQUE

Les dérivés de la présente invention ont été étudiés comparativement à la Buspirone, produit de référence connu à titre de ligand des récepteurs sérotoninergiques 5 $HT_{1A}$.

**A) Méthodologie :**

Les essais ont été réalisés sur des rats Wistar mâles de 200 à 220 g ayant libre accès à leur nourriture et à leur eau de boisson, dans des cages standards.

Les animaux sont isolés individuellement pour les essais d'hypothermie, de secrétion de corticostérone et de

position affaissée du corps (Flat Body Posture) ou réunis par groupes de trois pour le test des battements de la queue (Tail-Flicks).
La température du laboratoire est maintenue à 21 ± 1 °C sous une humidité de 60 ± 5 %. Ils sont soumis à un cycle lumière/obscurité de 12 heures/12 heures (le cycle lumière commençant à 7 h 30 du matin).

1) Etude in vitro - Test de Binding :

L'hippocampe issu des cerveaux de rats décapités a été immédiatement congélé sur glace carbonique puis conservé a - 80 °C jusqu'à la préparation des membranes. Le tissus a été homogénéisé à 4 °C dans le tampon approprié en utilisant un Polytron (Instruments Brinkman-Lucerne - Suisse) et centrifugé à 20.000 tours/mn.
L'incubation a été faite à 25 °C pendant 30 mn. La liaison non spécifique a été définie par 10 μmole de 5 HT. Les essais ont été terminés par filtration rapide à l'aide d'un collecteur de Brandel sur des filtres en fibre de verre prétraités avec 0,1 % de polyéthylène imine.
Pour chaque ligand froid, on a pris en compte un minimum de 3 valeurs produisant une inhibition entre 20 et 80 % de la liaison du ligand chaud. Les valeurs de concentrations inhibitrices 50 ($IC_{50}$) ont été déterminées selon le procédé 8 de Tallarida R.J et Murray R.B., Manual of Pharmacological calculations with computer programs, Springer Verlag, New York, (1987).
Le pKi a été calculé selon la formule :

$$pKi = -\log\left(\frac{IC_{50}}{1+[L]/Kd}\right)$$

dans laquelle [L] est la concentration du ligand chaud ([3]H-8-OH-DPAT, 0,4 nM) et Kd est la constante de dissociation apparente déterminée à partir des expériences de saturation.
Les substances étudiées ont été solubilisées dans le tampon d'incubation.

2) Etude in vivo

a/ Processus général concernant les tests d'activités agonistes et antagonistes sur les récepteurs $5HT_{1A}$.

Les composés à étudier ont été administrés par voie sous-cutanée (s.c) 60 minutes avant le début du test c'est à dire 30 minutes avant le solvant (réponses agonistes) ou le 8-OH-DPAT (réponses antagonistes).

Dans tous les essais le solvant est utilisé en parallèle comme contrôle. Les animaux ont été laissés au repos dans leur cage pendant le temps compris entre les injections et l'évaluation. Pour les études agonistes, le solvant a été administré à 1 ml/kg s.c. 30 minutes avant le début du test. Pour les études antagonistes, on a choisi des doses de 8-OH-DPAT induisant des réponses sous-maximales soit des doses de 0,63-0,16-0,16 et 0,16 mg/kg s.c. respectivement pour les tests de Tail-Flicks, Flat Body Posture, Sécrétion de Corticostérone et d'Hypothermie.

b/ Position affaissée du corps (Flat Body Posture ou FBP) et Sécrétion de Corticostérone (CS).

Les mêmes animaux ont été employés pour évaluer l'influence des composés étudiés sur le FBP et sur la détermination de la concentration plasmatique de CS. Tous les essais ont été réalisés le matin entre 10 h 30 et 12 h 30, soit lorsque les taux circadien de CS sont les plus faibles.

25 minutes après le traitement (soit 5 minutes avant la décapitation) les animaux sont observés dans leurs cages et on note la présence ou non de FBP.

La présence de FBP est définie par une position caractéristique de l'animal. Celui-ci est alors en position de decubitus ventral avec les membres postérieurs nettement en extension. 5 minutes après l'observation de FBP, les animaux sont décapités et le sang du tronc est recueilli dans des tubes refroidis contenant 50 μl d'une solution de EDTA à 10 % . Après centrifugation à 4000 tours/mn, le plasma est prélevé et conservé à - 30 °C jusqu'au dosage.

La CS a été déterminée en utilisant un dosage radio-compétitif pour une protéine plasmatique fixant la CS : la transcortine. Celle-ci est obtenue à partir d'un sérum de singe. La séparation des complexes CS-transcortine de la CS libre a été réalisée au moyen d'une solution de Dextran et de charbon actif. La limite de détection était de 50 pg/tube. Les variations de dosage intra- et inter-expériences étaient respectivement de 5 et 15 % [cf Rivet J.M. et al, Eur. J. Pharmacol., 183, 634-635 (1990)].

Les taux de base de CS dans le plasma n'étant jamais zéro, on a utilisé, pour calculer le pourcentage d'inhibition de CS plasmatique induite par 8-OH-DPAT, la formule suivante :

$$\% \text{ d'inhibition} = 100\times \frac{\text{(Antagoniste+Agoniste)- Antagoniste seul}}{\text{(Solvant+Agoniste)-Solvant seul}}$$

c/ Température corporelle (CT)

Les rats sont immobilisés et un thermomètre digital lubrifié (Thermistoprobe de Testotherm, Bale, suisse) est inséré dans le rectum à une profondeur de 5 cm. 30 secondes après l'insertion, la température est lue sur une échelle digitale. Le pourcentage d'inhibition est calculé à l'aide de la formule citée précédemment.

d/ Test spontané de Tail Flicks : (STF)

Les battements de la queue on été déterminés sur des animaux maintenus dans des cylindres en plastique opaque horizontaux, la queue des animaux pendant librement sur le bord de la paillasse de laboratoire. Après 5 minutes d'adaptation, on enregistre le nombre de mouvements émis en 5 minutes. Un STF est défini comme étant une élévation de la queue à un niveau supérieur à celui de l'axe du corps [Millan M.J. et al., J. Pharmacol. Exp. Ther., 256, 973-982 (1990)].

e/ Discrimination de drogue (DD)

Des rats mâles sont maintenus à 85 % de leur poids corporel normal par restriction alimentaire. Dans des chambres insonorisées, ces animaux sont entraînés à appuyer sur une pédale pour obtenir de la nourriture suivant une procédure FR10 (Fixed Ratio 10 ; la nourriture est délivrée au rat quand celui-ci a appuyé 10 fois sur le levier).

Acquisition ou entrainement

Des sessions quotidiennes de 15 minutes sont précédées par une injection de solvant ou de la drogue d'entraînement (0,31 mg/kg de 8-OH-DPAT, i.p.). L'animal reçoit une pilule de nourriture après 10 appuis sur la pédale "solvant" ou "drogue" suivant le composé administré avant la session.

Test de discrimination de drogue

Après la période d'acquisation, les animaux sont testés deux fois par semaine (mercredi et vendredi). Les trois autres jours, des sessions d'entraînement sont effectuées.

Les jours d'entraînement, les rats reçoivent soit du solvant soit du 8-OH-DPAT, 15 minutes avant d'être testé.

Les jours de test, les rats reçoivent la substance chimique 60 minutes avant la session.

La capacité pour la substance chimique administreé d'induire une réponse (appui sur le levier "solvant" ou "drogue" (8-OH-DPAT) est enregistrée.

Pour chaque substance et pour chaque dose, 7 animaux choisis au hasard sont utilisés. Pendant la période de test, le levier sur lequel le rat effectue les 10 premiers appuis est déterminé comme le levier choisi : une pilule de nourriture est alors délivrée.

Ce levier selectionné sera renforcé par la délivrance de nourriture jusqu'à la fin de la session de test. Le pourcentage d'animaux selectionnant le levier "drogue" à une dose particulière est calculé. De plus, le nombre total de réponses effectuées sur les deux leviers est exprimé en pourcentage du nombre de réponses effectuées sur le levier "solvant" obtenu lors d'une session d'entraînement précédente.

f/ analyse des résultats in vivo :

- En général, après analyse de variance, les résultats sont soumis au test de Dunett. Les résultats sont tenus pour significatifs si $p<0,05$.
  - Pour l'analyse des courbes dose-réponse concernant l'induction des STF, CS et Hypothermie, on a déterminé la dose efficace minimale (M.E.D) en mg/kg, c'est à dire la dose qui induit une réponse significativement différente de celle produite par le solvant.
  - Pour l'analyse des courbes dose-réponse concernant l'inhibition de STF, CS, et Hypothermie, les valeurs $ID_{50}$ -en mg/kg (dose réduisant de 50 % l'action de 8-OH-DPAT) ont été calculées ainsi que les limites de confiance à 95 % en utilisant une méthode inspirée de la méthode de Finney (1964).
  - Pour la dose-réponse d'induction et d'inhibition du FBP, les doses efficaces 50 ($ED_{50}$) (doses pour lesquelles 50 % des animaux montrent une réponse) ont été calculées par la méthode de Litchfield et Wilcoxon.

g/ Composés étudiés

Les doses des composés testés sont toutes exprimées en terme de base. Sauf mention contraire, tous les composés ont été dissous dans de l'eau stérile (additionnée si nécessaire de quelques gouttes d'acide lactique) et

administrés à un volume de 1 ml/kg s.c.

B) **Résultats :**

Les résultats sont regroupés dans les tableaux 1 et 2 ci-après.

Tableau 1

| **Binding des récepteurs 5 $HT_{1A}$** | |
|---|---|
| **MOLECULE** | **AFFINITE (pKi)** |
| Produit de référence BUSPIRONE | 7,93 |
| Exemple 1 | 8,76 |
| Exemple 3 | 8,77 |
| Exemple 4 | 9,42 |
| Exemple 5 | 8,89 |
| Exemple 6 | 8,99 |
| Exemple 7 | 9,23 |
| Exemple 10 | 8,92 |
| Exemple 11 | 8,77 |
| Exemple 12 | 8,96 |

Tableau 2

**Activité in vivo agoniste et antagoniste aux récepteurs 5-$HT_{1A}$**

| | STFs | | FBP | | CS Secretion | | HYPOTHERMIE | | DD 8-OH-DPAT | |
|---|---|---|---|---|---|---|---|---|---|---|
| | DME seule | $DI_{50}$ (95%LC) + DPAT | $DE_{50}$ (95%LC) seule | $DI_{50}$ (95%LC) + DPAT | DME seule | $DI_{50}$ (95%LC) + DPAT | DME seule | $DI_{50}$ (95%LC) + DPAT | $DE_{50}$ (95%LC) seule | $DI_{50}$ (95%LC) + DPAT |
| **Buspirone** | >40,0 | 3,7 (1,4-9,8) | 7,4 (2,2-25,6) | >10,0 | 2,5 | >10,0 | 2,5 | >10,0 | 1,35 (0,31-2,5) | >2,5 (0,63-2,5) |
| **% MPA** | | 86 | | 0 | | 0 | | 0 | | |
| **Exemple 1** | >10,0 | 0,12 (0,03-0,48) | >10,0 | 0,48 (0,19-1,25) | 10,0 | 0,43 (0,22-0,87) | 10,0 | 0,16 (0,05-0,53) | > 25 (2,5) | 0,63 (0,16-2,5) |
| **% MPA** | | 92 | | 100 | | 100 | | 98 | | 100 |
| **Exemple 3** | | <0,16 | <10,0 | 1,60 (0,46-5,58) | >10,0 | 1,56 (0,82-2,98) | >2,5 | 0,50 (0,17-1,46) | | |
| **% MPA** | | | | 100 | | 100 | | | | |
| **Exemple 5** | | | > 2,5 | < 2,5 | > 2,5 | 2,5 | >10,0 | 0,32 | | |
| **Exemple 10** | | | > 2,5 | < 2,5 | > 2,5 | 2,5 | >2,5 | 0,16 | | |
| **Exemple 11** | | | > 2,5 | < 2,5 | > 2,5 | 2,5 | >2,5 | 0,63 | | |

STFs = (Elevation spontanée de la queue)
FBP = (Position affaissée du corps)
CS = Corticostérone
DD = Discrimination de drogue
DME = Dose minimum efficace (versus témoin)
$DI_{50}$ = Dose inhibitrice (50 % d'inhibition)
$DE_{50}$ = Dose efficace (50 % d'effet)
95%LC = 95 % limite de confiance
%MPA = % d'antagonisme maximum possible

Les molécules ont été évaluées seules et contre 8-OH-DPAT (0,63 mg/kg, s.c.).

C) **Conclusion** :

L'examen des résultats repertoriés dans les tableaux 1 et 2 montre d'une part que les produits de l'invention ont une affinité pour le récepteur $5HT_{1A}$ nettement supérieure à celle de la buspirone et de plus que les composés de la présente invention ont un comportement antagoniste des récepteurs $5HT_{1A}$ contrairement à la buspirone qui, bien que se fixant également sur ces récepteurs, a un comportement agoniste.
D'où l'intérêt des composés de la présente invention dans le traitement des maladies du système nerveux central et des maladies neuroendocriniennes.

## Revendications

1. Pipérazines 1,4-disubstituées de formule (I) :

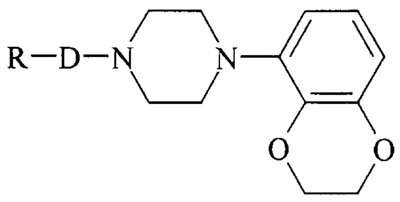

(I)

dans laquelle :
R représente un groupement choisi parmi :

- thiophén-3-yl et, dans ce cas, D représente un groupement choisi parmi méthylène, éthylène, n-propylène, n-butylène et 2,2-diméthyl-n-propylène,
- tétrahydrobenzo[b]thiophén-5-yl, cyclopenta[b]thiophén-5-yl et, dans ce cas, D représente le groupement méthylène,
- napht-1-yl, napht-2-yl, thiéno[2,3-b]thiophén-2-yl, thiophén-2-yl et, dans ce cas, D représente le groupement éthylène,
- et phényl et, dans ce cas, D représente le groupement n-propylène,

ainsi que leurs sels physiologiquement tolérables avec des acides appropriés.

2. Un composé de la revendication 1 qui est la : 4-(benzodioxan-5-yl)-1-[3-(thiophén-3-yl)propyl]pipérazine, et ses sels d'addition acides physiologiquement tolérables.

3. Un composé de la revendication 1 qui est la : 4-(benzodioxan-5-yl)-1-[(cyclopenta[b]thiophén-5-yl)méthyl]pipérazine, et ses sels d'addition acides physiologiquement tolérables.

4. Un composé de la revendication 1 qui est la : 4-(benzodioxan-5-yl)-1-[2-(napht-1-yl)éthyl]pipérazine, et ses sels d'addition acides physiologiquement tolérables.

5. Un composé de la revendication 1 qui est la : 4-(benzodioxan-5-yl)-1-[2-(napht-2-yl)éthyl]pipérazine, et ses sels d'addition acides physiologiquement tolérables.

6. Un composé de la revendication 1 qui est la : 4-(benzodioxan-5-yl)-1-[2-(thiéno[2,3-b]thiophén-2-yl)éthyl]pipérazine, et ses sels d'addition acides physiologiquement tolérables.

7. Un composé de la revendication 1 qui est la : 4-(benzodioxan-5-yl)-1-[2-(thiophén-3-yl)éthyl]pipérazine, et ses sels d'addition acides physiologiquement tolérables.

8. Un composé de la revendication 1 qui est la : 4-(benzodioxan-5-yl)-1-(3-phénylpropyl)pipérazine et ses sels d'addition acides physiologiquement tolérables.

9. Les compositions pharmaceutiques contenant comme principe actif un composé selon une des revendications 1 à 8 avec un ou plusieurs excipients pharmaceutiques appropriés.

**10.** Les compositions pharmaceutiques selon la revendication 9, présentées sous une forme convenant pour le traitement des maladies du système nerveux central et des maladies neuro-endocriniennes.

**Claims**

**1.** 1,4-Disubstituted piperazines of formula (I):

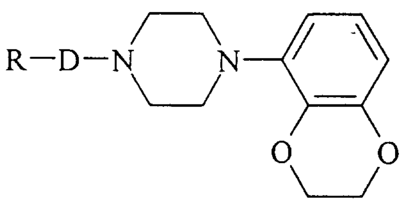

(I)

in which:
R represents a group selected from:

- thiophen-3-yl, and in that case D represents a group selected from methylene, ethylene, n-propylene, n-butylene and 2,2-dimethyl-n-propylene,
- tetrahydrobenzo[b]thiophen-5-yl, cyclopenta[b]thiophen-5-yl, and in those cases D represents a methylene group,
- naphth-1-yl, naphth-2-yl, thieno[2,3-b]thiophen-2-yl, thiophen-2-yl, and in those cases D represents an ethylene group,
- and phenyl, and in that case D represents an n-propylene group,

and their physiologically tolerable salts with suitable acids.

**2.** A compound according to claim 1 which is: 4-(benzodioxan-5-yl)-1-[3-(thiophen-3-yl)propyl]piperazine, and its physiologically tolerable acid addition salts.

**3.** A compound according to claim 1 which is: 4-(benzodioxan-5yl)-1-[(cyclopenta-[b]thiophen-5-yl)methyl]piperazine, and its physiologically tolerable acid addition salts.

**4.** A compound according to claim 1 which is: 4-(benzodioxan-5-yl)-1-[2-(naphth-1-yl)ethyl]piperazine, and its physiologically tolerable acid addition salts.

**5.** A compound according to claim 1 which is: 4-(benzodioxan-5-yl)-1-[2-(naphth-2-yl)ethyl]piperazine, and its physiologically tolerable acid addition salts.

**6.** A compound according to claim 1 which is: 4-(benzodioxan-5-yl)-1-[2(thieno-[2,3-b]thiophen-2-yl)ethyl]piperazine, and its physiologically tolerable acid addition salts.

**7.** A compound according to claim 1 which is: 4-(benzodioxan-5-yl)-1-[2-(thiophen-3-yl)ethyl]piperazine, and its physiologically tolerable acid addition salts.

**8.** A compound according to claim 1 which is: 4-(benzodioxan-5-yl)-1-(3-phenylpropyl)piperazine, and its physiologically tolerable acid addition salts.

**9.** Pharmaceutical compositions comprising as active ingredient a compound according to any one of claims 1 to 8 with one or more suitable pharmaceutical excipients.

**10.** Pharmaceutical compositions according to claim 9, presented in a form suitable for the treatment of diseases of the central nervous system and of neuroendocrine diseases.

## Patentansprüche

1. 1,4-Disubstituierte Piperazine der Formel (I):

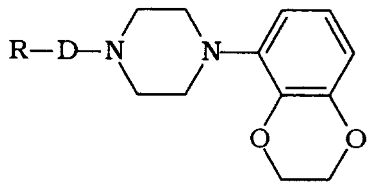

(I)

in der:
R eine Gruppe bedeutet ausgewählt aus:

- Thiophen-3-yl, wobei in diesem Fall D eine Gruppe ausgewählt aus Methylen, Ethylen, n-Propylen, n-Butylen und 2,2-Dimethyl-n-propylen darstellt,
- Tetrahydrobenzo[b]thiophen-5-yl, Cyclopenta[b]thiophen-5-yl, wobei in diesem Fall D die Methylengruppe darstellt,
- Naphth-1-yl, Naphth-2-yl, Thieno[2,3-b]thiophen-2-yl, Thiophen-2-yl, wobei in diesem Fall D die Ethylengruppe darstellt,
- und Phenyl, wobei in diesem Fall D die n-Propylengruppe darstellt,

sowie deren physiologisch verträgliche Salze mit geeigneten Säuren.

2. Verbindung nach Anspruch 1, nämlich: 4-(Benzodioxan-5-yl)-1-[3-(thiophen-3-yl)propyl]-piperazin und dessen Additionssalze mit physiologisch verträglichen Säuren.

3. Verbindung nach Anspruch 1, nämlich: 4-(Benzodioxan-5-yl)-1-[(cyclopenta[b]thiophen-5-yl)-methyl]-piperazin und dessen Additionssalze mit physiologisch verträglichen Säuren.

4. Verbindung nach Anspruch 1, nämlich: 4-(Benzodioxan-5-yl)-1-[2-(naphth-1-yl)-ethyl]-piperazin und dessen Additionssalze mit physiologisch verträglichen Säuren.

5. Verbindung nach Anspruch 1, nämlich: 4-(Benzodioxan-5-yl)-1-[2-(naphth-2-yl)-ethyl]-piperazin und dessen Additionssalze mit physiologisch verträglichen Säuren.

6. Verbindung nach Anspruch 1, nämlich: 4-(Benzodioxan-5-yl)-1-[2-(thieno[2,3-b]thiophen-2-yl)-ethyl]-piperazin und dessen Additionssalze mit physiologisch verträglichen Säuren.

7. Verbindung nach Anspruch 1, nämlich: 4-(Benzodioxan-5-yl)-1-[2-(thiophen-3-yl)-ethyl]-piperazin und dessen Additionssalze mit physiologisch verträglichen Säuren.

8. Verbindung nach Anspruch 1, nämlich: 4-(Benzodioxan-5-yl)-1-(3-phenylpropyl)-piperazin und dessen Additionssalze mit physiologisch verträglichen Säuren.

9. Pharmazeutische Zubereitungen enthaltend als Wirkstoff eine Verbindung nach einem der Ansprüche 1 bis 8 zusammen mit einem oder mehreren pharmazeutisch geeigneten Trägermaterialien.

10. Pharmazeutische Zubereitungen nach Anspruch 9 in Form einer für die Behandlung von Erkrankungen des Zentralnervensystems und von neuroendokrinen Erkrankungen geeigneten Form.